# EUROPEAN PATENT SPECIFICATION

(11) **EP 2 952 207 B1**
(45) Date of publication and mention of the grant of the patent: **27.03.2019**
(21) Application number: 15170455.8
(22) Date of filing: 03.06.2015
(51) Int. Cl.: A61K 47/02, A61K 47/14, A61K 9/10, A61K 31/4422, A61K 31/549

(54) **OIL BASED PHARMACEUTICAL COMPOSITIONS FOR ORAL ADMINISTRATION**
ÖLBASIERTE PHARMAZEUTISCHE ZUSAMMENSETZUNGEN ZUR ORALEN VERABREICHUNG
COMPOSITIONS PHARMACEUTIQUES POUR ADMINISTRATION ORALE À BASE D'HUILE

(30) Priority: 03.06.2014 GB 201409869
(43) Date of publication of application: 09.12.2015
(73) Proprietor: Essential Pharmaceuticals Ltd, Egham, Surrey TW20 8RB (GB)
(72) Inventor: Engineer, Nikesh, Egham, Surrey TW20 8RB (GB)
(74) Representative: Elsworth, Jon David

(56) References cited:
- EP-A1- 0 273 890
- EP-A1- 0 760 237
- EP-A2- 0 389 177
- WO-A1-92/04893
- WO-A1-96/06599
- WO-A1-2006/008640
- WO-A2-2008/039472
- AU-B4- 2010 101 089
- US-A- 5 795 902
- "Amlodipine besylate 1 mg/mL veterinary suspension", INTERNATIONAL JOURNAL OF PHARMACEUTICAL COMPOUNDING, IJPC, EDMOND, OK, US, vol. 13, no. 5, 1 September 2009 (2009-09-01), page 429, XP009185669, ISSN: 1092-4221
- CHHABRA G ET AL: "Design and development of nanoemulsion drug delivery system of amlodipine besilate for improvement of oral bioavailability", DRUG DEVELOPMENT AND INDUSTRIAL PHARMACY, NEW YORK, NY, US, vol. 37, no. 8, 1 August 2011 (2011-08-01) , pages 907-916, XP009185664, ISSN: 0363-9045, DOI: 10.3109/03639045.2010.550050 [retrieved on 2011-03-14]
- UK Medicines Information (UKMi) pharmacists: "Q&A 294.3 - What are the therapeutic options for patients unable to take solid oral dosage forms", Medicines Q&A, NHS evidence search , July 2013 (2013-07), XP002742988, Retrieved from the Internet: URL:www.elmmb.nhs.uk/EasySiteWeb/GatewayLi nk.aspx?alId=52779 [retrieved on 2015-08-04]

## Description

The present invention relates to a pharmaceutical composition. In particular, it concerns a pharmaceutical composition suitable for the oral administration of drug compounds which are typically unstable in aqueous environments, and/or possess an unappealing taste or sensation when swallowed.

It is estimated that about as much as 40% of all active substances identified by combinatorial screening methods are challenging to formulate using conventional methodologies. This is typically due to factors such as low drug stability in the physical environment. In particular, where a particular route of administration is favoured for a given active agent, e.g. for administration to a specific patient population, such as children, it can often be difficult to prepare an appropriate formulation.

To overcome such problems for active agents intended for oral administration, a number of strategies have been developed, including tablet, capsule, and liquid dosage systems. For active agents which are unstable in aqueous environments, liquid formulations typically involve preparing oral emulsions (e.g. oil-in-water) of the active agent solubilised in an appropriate solvent system. For example, US5929030 describes pharmaceutical compositions in which drug compounds are completely dissolved in an oil-based solvent system and then combined with an aqueous solvent to form an oil-in-water oral microemulsion.

Alternatively, active agents may be suspended in an appropriate solvent system using stabilising components such as surfactants. For example, US2005/0009908 describes aqueous-based formulations in which a drug nanoparticle is suspended in an aqueous solvent and stabilised by coating the nanoparticle with an oil-based solvent and various surfactants.

Oil-based suspensions have also been identified for injectable preparations. WO2012/095438 describes oil-based suspensions comprising crystalline cephalosporin drug compounds which have been uniformly crystallised by means of a diafiltration method (cross-flow filtration) employing membrane filters.

An oil-based veterinary suspension of amlodipine besylate is known from International Journal of Pharmaceutical Compounding, Vol. 13 No. 5, 2009.

However, there is still a commercial need for pharmaceutical formulations in which a variety of active agents can be made stable in a liquid environment, and stored for extended periods of time. In particular, prior art formulations do not provide a simple means by which to deliver active agents in particulate form having a reduced stability in the presence of aqueous media. Furthermore, the prior art does not provide a composition which can also be used to deliver active agents which are unpalatable or elicit an uncomfortable sensation in the mouth and/or throat (e.g. burning sensation) when swallowed. These factors are particularly important for liquid compositions intended for administration to children.

Accordingly, in a first aspect of the invention, there is provided a pourable pharmaceutical composition suitable for oral administration, wherein the composition comprises at least one pharmaceutically active agent in micronised form, dispersed within a liquid phase which comprises at least one triglyceride of one or more medium-chain fatty acid residues, an anti-caking agent, and up to 2% w/w water, based on the overall composition, wherein the at least one micronised pharmaceutically active agent is bendroflumethiazide or amlodipine, or a pharmaceutically acceptable salt thereof.

Surprisingly, it has been found that compositions of this type can be used to formulate active agents which are otherwise unstable in liquid environments, especially in the presence of water. The compositions exhibit excellent storage stability in accordance with ICH (International Conference on Harmonisation of Technical Requirements for Registration of Pharmaceuticals for Human Use) guidelines. In addition, the compositions are appropriate for active agents which are poorly soluble or entirely insoluble in water.

The terms "active agent," "drug" and "pharmaceutically active agent" as used herein refer to a chemical material or compound which, when administered to an organism (human or animal, preferably human), induces a desired pharmacological effect. Included in the meaning of these terms are derivatives of the pharmaceutically active agent such as pharmaceutically acceptable salts.

The terminology "% w/w" as used herein refers to the relative amount of a component in terms of weight, based on the overall composition.

The term "short-chain fatty acid residue" as used herein refers to linear or branched, saturated or unsaturated aliphatic carboxylic acid residues having a carbon backbone of 1 to 5 carbon atoms. Suitable short-chain fatty acid residues include: formic acid; acetic acid; propionic acid; butyric (butanoic) acid; isobutyric (2-methylbutanoic) acid; valeric (pentanoic) acid; and isovaleric (3-methylbutanoic).

The term "medium-chain fatty acid residue" as used herein refers to linear or branched, saturated or unsaturated (preferably saturated) aliphatic carboxylic acid residues having a carbon backbone of 6 to 12 carbon atoms. Suitable medium-chain fatty acid residues include: caproic (hexanoic) acid; caprylic (octanoic) acid; capric (decanoic) acid; and lauric (dodecanoic) acid. Preferably, the medium-chain fatty acid residue refers to caprylic (octanoic) acid and/or capric (decanoic) acid.

The term "long-chain fatty acid residue" as used herein refers to linear or branched, saturated or unsaturated aliphatic carboxylic acid residues having a carbon backbone of 13 to 21 carbon atoms. Suitable long-chain fatty acid residues include: myristic (tetradecanoic) acid; palmitic (hexadecanoic) acid; stearic (octadecanoic) acid; linoleic acid; and arachidic (eicosanoic) acid.

The term "refined natural lipid oils" as used herein refers to oils isolated/extracted from natural sources which have been refined to remove impurities such as allergens. Such refined oils may also be bleached and/or deodorised to remove any colouration and/or aroma associated with the natural source. Refined oils may also be processed further by hydrogenation to at least partially reduce the unsaturation in any unsaturated fats/oils which are present. In addition, such oils may be fractionated to provide an oil having a defined range of components, i.e. a specific fraction of the original oil, such as an oil containing only medium-chain fatty acid derivatives. Examples of refined natural lipid oils include coconut oil, castor oil, palm kernel oil, and palm oil (preferably coconut oil).

The liquid phase comprises up to 2% w/w water, based on the overall composition. Most preferably, the liquid phase comprises essentially no water, or no detectable amount of water, as determined by the well-known Karl Fischer method. Such low levels of water are advantageous from a stability perspective, since it significantly reduces the likelihood of degradation of the active agent and, in turn, the formation of undesirable impurities. It is also beneficial from a regulatory perspective, since it reduces the number of components in the composition, particularly the need for preservatives, emulsifiers, solubilisers and surfactants that are typically required for aqueous-based compositions.

The water in the liquid phase may be free water or water bound to another component of the liquid phase, or a combination of both. For example, bound water may be water held in close association with another component of the liquid phase such as by electrostatic forces. Preferably, the above preferred embodiments specifying upper limits of water refer to the amount of free water in the pharmaceutical composition, since it is free water which poses a potential stability problem for the active agent.

Also in relation to improved stability, it is preferable that the liquid phase of the pharmaceutical composition is present as a single phase. This is particularly advantageous in embodiments in which there is a low level of water in the composition, such as less than 2% w/w water, based on the overall composition, or where essentially no water is present in the composition and/or where no water has been added to the composition during production. The amount of active agent in the pharmaceutical composition is not particularly limited. It is preferable, nonetheless, that the at least one pharmaceutically active agent is present in a concentration of 0.001% w/w to 5% w/w, based on the overall composition. More preferably, for optimal stability of the composition, the at least one pharmaceutically active agent is present in a concentration of 0.1% w/w to 1% w/w, based on the overall composition.

The liquid phase comprises at least one triglyceride having one or more medium-chain fatty acid residues. For example, triglycerides of medium-chain fatty acid residues (preferably linear, saturated aliphatic carboxylic acid residues having a carbon backbone of 6 to 12 carbon atoms, more preferably 8 to 10 carbon atoms, e.g. CremerCOOR® MCT 60/40) have been found to be surprisingly advantageous with regard to the long-term storage stability of the composition, and particularly effective in terms of the bioavailability of the active agent. Triglycerides having one or more short-, medium-, or long-chain fatty acid residues may also be preferred.

The oil component constitutes the major component of the pharmaceutical composition and may be present in a concentration range of 1% w/w to 99% w/w, based on the overall composition. Preferably, it is present in a concentration range of 10% w/w to 99% w/w, 50% w/w to 99% w/w, 70% w/w to 98% w/w, or 80% w/w to 98% w/w, based on the overall composition.

In another embodiment, the liquid phase further comprises one or more hydrophilic liquid components selected from glycerine, α-propylene glycol, β-propylene glycol, and polyethylene glycol. The hydrophilic liquid component may also be present in a concentration range of 1% w/w to 99% w/w, preferably 10% w/w to 99% w/w, 10% w/w to 70% w/w, or 10% w/w to 50% w/w, based on the overall composition.

The active agent of the composition is present in a particulate form, and is dispersed (e.g. suspended) within the oil. The particulate form can be crystalline or amorphous. The particulate form of the active agent is micronised to a desired particle size distribution before incorporation in the composition. Micronisation allows the particle size distribution to be controlled such that the active agent is optimally dispersed within the oil. This facilitates the preparation of a substantially homogenous liquid dispersion.

In a preferred embodiment, the active agent has an average particle size diameter in the range of 2 nm to 1 µm, such as 10 nm to 0.9 µm or 20 nm to 0.8 µm. Most preferably, the active agent has an average particle size diameter in the range of 100 nm to 0.5 µm. The average particle size diameter preferably relates to the median particle size diameter, e.g. D50, as determined by laser diffraction methods.

Active ingredients for incorporation in the composition can be selected from a variety of known classes of drugs or diagnostic agents, provided they are suitable for oral administration and dispersion in the liquid phase. Active agents which are poorly soluble in aqueous media are favoured, along with active agents possessing an unappealing taste and/or uncomfortable sensation when swallowed.

The expression "poorly soluble" as used herein refers to compounds which are not soluble in ≤ 250 mL of aqueous media over the range pH 1 to pH 7.5. This is in accordance with the Biopharmaceutical Classification System (BCS).

According to the invention, the at least one pharmaceutically active agent is either bendroflumethiazide or amlodipine, or a pharmaceutically acceptable salt thereof.

The pharmaceutical composition may also comprise one or more pharmaceutically acceptable excipients. The term "excipients" as used herein refers to substances other than the pharmaceutically active agent which are included in the manufacturing process or are contained in the finished pharmaceutical product dosage form. Excipients are classified by the functions they perform in the pharmaceutical dosage form. For example, the composition may contain one or more sweetening agents, anti-caking agents, surface active agents (e.g. surfactants), viscosity-controlling agents, and/or anti-oxidants.

The composition may contain one or more sweetening agents for the purposes of making the composition more palatable. Suitable sweetening agents include liquid components based on sucralose, fructose, sorbitol, maltitol, aspartame, cyclamate, and saccharin. In terms of taste in the pharmaceutical composition of the invention, and overall stability, sucralose is a particularly preferred sweetening agent. Sweetening agents may be present in the composition in an amount of up to 10% w/w, based on the overall composition, preferably up to 5% w/w.

The composition comprises one or more anti-caking agents. Suitable anti-caking agents include magnesium trisilicate, sodium aluminosilicate, calcium aluminosilicate, polydimethylsiloxanes, and colloidal silicon dioxides. In a preferred embodiment, the anti-caking agent is one or more colloidal silicon dioxides, particularly anhydrous colloidal silicon dioxides (e.g. Aerosil 200 and/or Aerosil 972, preferably Aerosil 200).

When the composition comprises essentially no water or no detectable amount of water, the composition preferably comprises colloidal silicon dioxide. This is because the colloidal silicon dioxide not only helps to provide a substantially homogenous liquid composition of active agent, but is also particularly effective as a viscosity-increasing agent and as a desiccant, i.e. it absorbs residual amounts of water in the composition.

The composition may also comprise one or more surface active agents, e.g. surfactants. Suitable surface active agents include anionic, cationic, zwitterionic (ampholytic) and non-ionic surfactants. Preferably, the surface active agent is a non-ionic surfactant, such as polyoxyethylene hydrogenated castor oils type 40 and/or 60 and polysorbate 80. Such surfactants are effective in stabilising the dispersion of the one or more active agents, and may be useful for providing a homogeneous dispersion (i.e. single phase) if the amount of free water in the pharmaceutical composition exceeds trace levels, 2% w/w, 5% w/w, or 10% w/w, based on the overall composition.

The anti-caking agent and the surfactant may be present in the composition in an amount of up to 10% w/w, based on the overall composition, preferably up to 5% w/w.

In one preferred embodiment, the composition may comprise both an anti-caking agent and a surfactant. However, it has been surprisingly found that the stability of the composition is optimal in the absence of a surfactant.

Viscosity-controlling agents may also be present in the composition, where appropriate, in order to provide the desired rheology. Suitable viscosity-controlling agents include colloidal silicon dioxides, methyl-ethyl-propyl cellulose, ethyl cellulose, polyethyleneglycols, or mixtures thereof. Preferably, the viscosity-controlling agent is a polyethyleneglycol. Viscosity-controlling agents may be present in the composition in an amount of up to 5% w/w, preferably in the range of 0.05% w/w to 3% w/w.

Stabilising or anti-oxidant agents, or combinations thereof, either from natural sources or chemical/artificial sources, may also be present in the pharmaceutical composition of the invention. Suitable agents include tocoferols, tocotrienols, retinol, retinal, and retinoic acid. Lipophilic anti-oxidant agents selected from the above are preferred, such as tocoferols. These agents may be present in the composition in an amount of up to 10% w/w, preferably in the range of 0.05% w/w to 8% w/w.

In a particularly preferred embodiment of the first aspect, the pourable pharmaceutical composition comprises at least one micronised pharmaceutically active agent, suspended within a liquid phase which comprises (preferable consists of) a triglyceride having one or more medium chain fatty acids (preferably a triglyceride of C₆₋₁₂ fatty acids, particularly C₈₋₁₀ fatty acids such as capric and caprylic fatty acids), a sweetening agent (preferably sucralose), and an anti-caking agent (preferably colloidal silicon dioxide), and wherein the composition comprises essential no water (preferably no detectable amount of water). In this embodiment, it is also preferable that the composition does not contain a surfactant.

In a second aspect of the invention, there is provided a pharmaceutical composition according to the first aspect, for use in therapy.

In a third aspect of the invention, there is provided a process for preparing a pharmaceutical composition according to the first aspect, the process comprising providing a liquid phase which comprises one or more oils selected from triglycerides having one or more medium-chain fatty acids, and refined natural lipid oils, and up to 2% w/w water, based on the overall composition, and homogeneously dispersing the at least one pharmaceutically active agent in particulate form in the liquid phase. In particular, the one or more active agents are micronised to the desired particle size distribution and homogeneously suspended, by means of conventional techniques, in the liquid phase.

It will be appreciated that the preferred embodiments of the first aspect of the invention also apply to the second and third aspects.

The invention will now be described in more detail by way of example only.

### Examples

Unless indicated otherwise, parts are represented as parts by weight (w/w), temperature is represented in degrees centigrade and pressure is at or near atmospheric pressure. All components were obtained from commercial sources unless noted otherwise.

### Comparative Example 1 - Bendroflumethiazide aqueous suspensions

Table 1 illustrates the results of an initial analysis on a bendroflumethiazide 2.5 mg/5 mL aqueous suspension batch against a placebo batch. Analysis was performed in accordance with the relevant pharmacopeial monograph.

**Table 1 - Initial analysis of aqueous suspensions**

| | Bendroflumethiazide 2.5 mg/5 mL suspension batch | Bendroflumethiazide 2.5 mg/5 mL placebo suspension batch |
|---|---|---|
| pH | 4.65 | 4.63 |
| Density (g/mL) | 1.0071 | 1.0065 |
| Bendroflumethiazide (mg/5 mL) | 2.48 | N.D. |
| Bendroflumethiazide impurity A | 8% area of API peak | N.D. |
| Altizide | N.D. | N.D. |
| Sodium benzoate (mg/5 mL) | 10.77 | 9.81 |

| | | |
|---|---|---|
| N.D. = Not detected Bendroflumethiazide impurity A = 4-amino-6-(trifluoromethyl)benzene-1,3-disulfonamide | | |

The initial analysis clearly indicated an increased concentration of the bendroflumethiazide impurity A at a suspension pH level of ± 4.6. Given that literature studies have indicated that aqueous bendroflumethiazide suspensions set at a more acidic level can be beneficial to limit the increase of impurity A, further studies were conducted at pH levels of 3 and 5 in order to see if any improvements could be made. Tables 2 to 6 show the results from a stability study on such suspension samples stored at 25°C/60%RH, 40°C/75%RH and 5°C (RH = relative humidity).

**Table 2 - Bendroflumethiazide aqueous suspension stability at pH 3, stored at 25°C/60%RH**

| | t=1 week | t=2 weeks | t=3 weeks | t=4 weeks | t=6 weeks | t=9 weeks | t=12 weeks |
|---|---|---|---|---|---|---|---|
| Bendroflumethiazide (mg/5 mL) | 2.19 | 2.29 | 2.15 | 2.13 | 2.08 | 2.0 | 1.95 |
| Bendroflumethiazide impurity A (% relative to API) | N.D. | 0.59 | 1.20 | 2.05 | 2.32 | 3.80 | 4.84 |
| Sodium benzoate (mg/5 mL) | 9.75 | 9.70 | 9.81 | 9.75 | 9.64 | 9.73 | 9.71 |
| pH | 3.0 | | 3.1 | | | | |

**Table 3 - Bendroflumethiazide aqueous suspension stability at pH 3, stored at 40°C/75%RH**

| | t=1 week | t=2 weeks | t=3 weeks | t=4 weeks |
|---|---|---|---|---|
| Bendroflumethiazide (mg/5 mL) | 2.19 | 1.90 | 1.80 | 1.58 |
| Bendroflumethiazide impurity A (% relative to API) | N.D. | 6.40 | 12.06 | 18.16 |
| Sodium benzoate (mg/5 mL) | 9.75 | 9.69 | 9.82 | 9.70 |
| pH | 3.0 | | 3.1 | |

**Table 3 - Bendroflumethiazide aqueous suspension stability at pH 3, stored at 5°C**

| | t=1 week | t=2 weeks | t=3 weeks | t=4 weeks | t=12 weeks |
|---|---|---|---|---|---|
| Bendroflumethiazide (mg/5 mL) | 2.19 | 2.29 | 2.15 | 2.13 | 2.12 |
| Bendroflumethiazide impurity A (% relative to API) | N.D. | 0.59 | 1.20 | 2.05 | 9.71 |
| Sodium benzoate (mg/5 mL) | 9.75 | 9.70 | 9.81 | 9.75 | |
| pH | 3.0 | | 3.1 | | 2.0 |

**Table 5 -Bendroflumethiazide aqueous suspension stability at pH 5, stored at 25°C/60%RH**

| | t=1 week | t=2 weeks | t=3 weeks | t=4 weeks |
|---|---|---|---|---|
| Bendroflumethiazide (mg/5 mL) | 2.28 | 1.85 | 1.75 | 1.71 |
| Bendroflumethiazide impurity A (% relative to API) | 0.89 | 8.10 | 12.17 | 13.13 |
| Sodium benzoate (mg/5 mL) | 10.18 | 10.05 | 10.09 | 10.02 |
| pH | 5.0 | | 4.9 | |

**Table 5 -Bendroflumethiazide aqueous suspension stability at pH 5, stored at 40°C/75%RH**

| | t=1 week | t=2 weeks | t=3 weeks | t=4 weeks |
|---|---|---|---|---|
| Bendroflumethiazide (mg/5 mL) | 2.28 | 1.48 | 1.35 | 1.26 |
| Bendroflumethiazide impurity A (% relative to API) | 0.89 | 22.20 | 28.60 | 30.91 |
| Sodium benzoate (mg/5 mL) | 10.18 | 1.09 | 10.27 | 10.04 |
| pH | 5.0 | | 5.0 | |

The results show that bendroflumethiazide impurity A concentrations were observed in all investigated samples, at all storage conditions and pH levels. The impurity A concentration increased during the stability study from the initial concentration in the prepared batch. As a result, it is clear that aqueous suspensions are unsuitable for the preparation and storage of such active agents.

### Example 1 - Formulation for a Multi-dose Amlodipine 5mg/5mL lipophilic suspension

### Preparation:

In a pharmaceutical manufacturing process, 13.86 mg of amlodipine besylate (equal to 5 mg amlodipine), comprising 0.15% by weight, was homogenised in 9,350 mg medium-chain triglycerides, comprising 98.8% by weight. Subsequently, colloidal silicon dioxide 100mg, comprising 1% by weight, was added. The homogenised suspension was analysed to be stable and filled into amber glass bottles.

| **Component** | **Quantity (mg/5ml)** |
|---|---|
| Amlodipine besylate micronised | 6.93 |
| Medium-chain triglyceride (COOR MCT 60/40 EP) | 4.675 |
| Sucralose | 1.25 |
| Colloidal Silicon Dioxide (Aerosil 200) | 5.0 |

### Example 2 - Stability Studies of Multi-dose Amlodipine 5mg/5mL lipophilic suspension

To evaluate the stability of the amlodipine 5mg/5ml suspension of Example 1 in an indicative 3 months stability and in-use stability study, a lab scale batch of the composition was manufactured. The batch was filled in 150ml Amber (type III) glass bottles with a child resistant HDPE/LDPE closure, stored and analysed accordingly.

### Study conditions:

The amlodipine 5mg/5ml batch was stored for 3 months at ICH storage conditions 25°C/60%RH and analysed. The in-use stability study was performed according to a pre-defined sampling schedule on amlodipine 5mg/5ml samples stored at 25°C/60%RH.

### 2.1 Indicative 3 months stability study

Analysis was performed with validated analytical methods allowing compliance to pre-defined product specifications and limits for: amlodipine assay, related substances (impurities), suspension appearance, HPLC & UV identity and 20°C relative density. The samples pull point schedule for the 3 months stability study for ICH 25°C/60%RH condition was set at t=0 and t=3months.
Results: Samples stored at 25°C/60%RH complied with amlodipine 5mg/5ml analytical product specifications and limits.

### 2.2 In-use stability study

The 29 days in-use stability study was performed on amlodipine 5mg/5ml samples stored at 25°C/60%RH in month 3 of the study. Analysis was performed with validated methods according to pre-defined product specifications and limits for: amlodipine assay, related substances (impurities), HPLC and UV identity, 20°C relative density and closure integrity of the bottle cap.

In-use study sampling instructions: take 5mL of amlodipine 5mg/5ml suspension each day with a preliminary plastic pipette, pull point sample analysis was set at t=0, t=10 days, t=20 days and t=29 days.

The bottle closure integrity and amlodipine 5mg/5ml suspension appearance was tested each day for a period of 29 days. Bottle closure integrity was tested by turning the bottle upside down for 10 seconds and a visual check if no leaking occurs.
Results: Analysed results complied with amlodipine 5mg/5ml product specifications and limits.

### 2.3 Physical Compatibility Study

A 3 months compatibility study was performed on the amlodipine 5mg/5ml suspension. Test article solutions were prepared on a lab scale, packed in amber sample bottles, and stored in the dark at room temperature. The following test article solutions were prepared for evaluation:
a) Amlodipine 5mg/5ml suspension according to Example 1
b) Oil suspension of all inactive ingredients (i.e. containing no amlodipine)
Test parameters and results: Amlodipine 5mg/5ml suspensions were evaluated on appearance of the suspension. The amlodipine 5mg/5ml suspensions were observed and remained unchanged in the 3 months investigational study.

### Example 3 - Formulation for a Multi-dose Bendroflumethiazide 0.5mg/mL lipophilic suspension

### Preparation:

In a pharmaceutical manufacturing process, 0.5 mg of bendroflumethiazide, comprising 0.05% by weight, was homogenised in 9,233mg medium-chain triglycerides, comprising 98.3% by weight. Subsequently, colloidal silicon dioxide 150mg, comprising 1.6% by weight, was added. The homogenised suspension was analysed to be stable and filled into amber glass bottles.

| **Component** | **Quantity (mg/5ml)** |
|---|---|
| Bendroflumethiazide micronised | 2.5 |
| Medium-chain triglyceride (COOR MCT 60/40 EP) | 4.617 |
| Sucralose | 1.30 |
| Colloidal Silicon Dioxide (Aerosil 200) | 7.50 |

### Example 4 - Stability Studies of Multi-dose Bendroflumethiazide 2.5mg/5mL lipophilic suspension

To evaluate the stability of the bendroflumethiazide 2.5mg/5ml formulation in an indicative 3 months stability and in-use stability study, a lab scale batch of the composition according to Example 3 was manufactured. The batch was filled in 150ml Amber (type III) glass bottles with a child resistant HDPE/LDPE closure, stored and analysed accordingly.

### Study conditions:

The bendroflumethiazide 2.5mg/5ml batch was stored for 3 months at ICH storage conditions 25°C/60%RH and analysed. The in-use stability study was performed according to a pre-defined sampling schedule on bendroflumethiazide 2.5mg/5ml samples stored at 25°C/60%RH.

### 4.1 Indicative 3 months stability study

Analysis was performed with validated analytical methods allowing compliance to pre-defined product specifications and limits for: bendroflumethiazide assay, related substances (impurities), suspension appearance, HPLC identity and 20°C relative density. The samples pull point schedule for the 3 months stability study for ICH 25°C/60%RH condition was set at t=0 and t=3months.
Results: Samples stored at 25°C/60%RH complied with bendroflumethiazide 2.5mg/5ml analytical product specifications and limits.

### 4.2 In-use stability study

The 29 days in-use stability study was performed on bendroflumethiazide 2.5mg/5ml samples stored at 25°C/60%RH in month 3 of the study and analysed.

Analysis was performed with validated methods according to pre-defined product specifications and limits for: bendroflumethiazide assay, related substances (impurities), HPLC identity, 20°C relative density and closure integrity of the bottle cap.

In-use study sampling instructions: take 5mL of bendroflumethiazide 2.5mg/5ml suspension each day with a preliminary plastic pipette, pull point sample analysis was set at t=0, t=10 days, t=20 days and t=29 days.

The bottle closure integrity and bendroflumethiazide 2.5mg/5ml suspension appearance was tested each day for a period of 29 days. Bottle closure integrity was tested by turning the bottle upside down for 10 seconds and a visual check if no leaking occurs
Results: Analysed results complied with bendroflumethiazide 2.5mg/5ml product specifications and limits.

### 4.3 Physical Compatibility Study

A 3 months compatibility study was performed on the bendroflumethiazide 2.5mg/5ml suspension. Test article solutions were prepared on a lab scale, packed in amber sample bottles, and stored in the dark at room temperature. The following test article solutions were prepared for evaluation:
a) Bendroflumethiazide 2.5mg/5ml suspension
b) Oil suspension of all inactive ingredients (i.e. containing no bendroflumethiazide)
Test parameters and results: Bendroflumethiazide 2.5mg/5ml suspensions were evaluated on appearance of the suspension. The bendroflumethiazide 2.5mg/5ml suspensions were observed and remained unchanged in the 3 months investigational study.

## Claims

1. A pourable pharmaceutical composition suitable for oral administration, wherein the composition comprises at least one pharmaceutically active agent in micronised form, dispersed within a liquid phase which comprises at least one triglyceride of one or more medium-chain fatty acid residues, an anti-caking agent, and up to 2% w/w water, based on the overall composition, wherein the at least one micronised pharmaceutically active agent is bendroflumethiazide or amlodipine, or a pharmaceutically acceptable salt thereof.

2. The pharmaceutical composition according to claim 1, wherein the liquid phase comprises essentially no water.

3. The pharmaceutical composition according to claim 1 or claim 2, wherein the liquid phase is present as a single phase.

4. The pharmaceutical composition according to any preceding claim, wherein the at least one pharmaceutically active agent is present in a concentration of 0.001% w/w to 5% w/w, based on the overall composition, such as wherein the at least one pharmaceutically active agent is present in a concentration of 0.1% w/w to 1% w/w, based on the overall composition.

5. The pharmaceutical composition according to any preceding claim, wherein the liquid phase further comprises one or more hydrophilic liquid components selected from glycerine, α-propylene glycol, β-propylene glycol, and polyethylene glycol.

6. The pharmaceutical composition according to any preceding claim, wherein the at least one triglyceride of one or more medium-chain fatty acid residues is present in a concentration range of 1% w/w to 99% w/w, based on the overall composition, such as 10% w/w to 99% w/w, based on the overall composition.

7. The pharmaceutical composition according to claim 5 or claim 6, wherein the hydrophilic liquid component is present in a concentration range of 10% w/w to 99% w/w, based on the overall composition.

8. The pharmaceutical composition according to any preceding claim, wherein the at least one pharmaceutically active agent has a particle size diameter in the range of 2 nm to 1 µm, such as wherein the at least one pharmaceutically active agent has a particle size diameter in the range of 100 nm to 0.5 µm.

9. The pharmaceutical composition according to any preceding claim, wherein the anti-caking agent is colloidal silicon dioxide.

10. A pharmaceutical composition according to any preceding claim, for use in therapy.

11. A process for preparing a pharmaceutical composition according to any one of claims 1 to 9, the process comprising providing a liquid phase which comprises at least one triglyceride of one or more medium-chain fatty acid residues, an anti-caking agent, and up to 2% w/w water, based on the overall composition, and homogeneously dispersing the at least one pharmaceutically active agent in micronised form in the liquid phase, wherein the at least one micronised pharmaceutically active agent is bendroflumethiazide or amlodipine, or a pharmaceutically acceptable salt thereof.

## Patentansprüche

1. Fließfähige pharmazeutische Zusammensetzung zur oralen Verabreichung, wobei die Zusammensetzung zumindest einen pharmazeutischen Wirkstoff in mikronisierter Form enthält, der in einer flüssigen Phase dispergiert ist, die zumindest ein Triglycerid aus einem oder mehreren mittelkettigen Fettsäureresten, ein Antiklumpmittel und bis zu 2 Gew.-% Wasser, bezogen auf die Gesamtzusammensetzung, enthält, wobei der mindestens eine mikronisierte pharmazeutische Wirkstoff Bendroflumethiazid oder Amlodipin oder ein pharmazeutisch akzeptables Salz davon ist.

2. Pharmazeutische Zusammensetzung nach Anspruch 1, wobei die flüssige Phase im Wesentlichen kein Wasser enthält.

3. Pharmazeutische Zusammensetzung nach Anspruch 1 oder Anspruch 2, wobei die flüssige Phase als Einzelphase vorliegt.

4. Pharmazeutische Zusammensetzung nach einem der vorhergehenden Ansprüche, wobei der mindestens eine pharmazeutische Wirkstoff in einer Konzentration von 0,001 Gew.-% bis 5 Gew.-%, bezogen auf die Gesamtzusammensetzung, beispielsweise in einer Konzentration von 0,1 Gew.-% bis 1 Gew.-%, bezogen auf die Gesamtzusammensetzung, vorliegt.

5. Pharmazeutische Zusammensetzung nach einem der vorhergehenden Ansprüche, wobei die flüssige Phase ferner eine oder mehrere hydrophile flüssige Bestandteile enthält, die ausgewählt sind aus Glycerin, α-Propylenglykol, β-Propylenglykol und Polyethylenglykol.

6. Pharmazeutische Zusammensetzung nach einem der vorhergehenden Ansprüche, wobei das mindestens eine Triglycerid aus einem oder mehreren mittelkettigen Fettsäureresten in einem Konzentrationsbereich von 1 Gew.-% bis 99 Gew.-%, bezogen auf die Gesamtzusammensetzung, beispielweise 10 Gew.-% bis 99 Gew.-%, bezogen auf die Gesamtzusammensetzung, vorliegt.

7. Pharmazeutische Zusammensetzung nach Anspruch 5 oder Anspruch 6, wobei der hydrophile flüssige Bestandteil in einem Konzentrationsbereich von 10 Gew.-% bis 99 Gew.-%, bezogen auf die Gesamtzusammensetzung, vorliegt.

8. Pharmazeutische Zusammensetzung nach einem der vorhergehenden Ansprüche, wobei der mindestens eine pharmazeutische Wirkstoff einen Teilchengrößendurchmesser in dem Bereich von 2 nm bis 1 µm, beispielsweise in dem Bereich von 100 nm bis 0,5 µm aufweist.

9. Pharmazeutische Zusammensetzung nach einem der vorhergehenden Ansprüche, wobei das Antiklumpmittel kolloidales Siliziumdioxid ist.

10. Pharmazeutische Zusammensetzung nach einem der vorhergehenden Ansprüche zur Verwendung in der Therapie.

11. Verfahren zur Herstellung einer pharmazeutischen Zusammensetzung nach einem der Ansprüche 1 bis 9, wobei das Verfahren umfasst: das Bereitstellen einer flüssigen Phase, die mindestens ein Triglycerid aus einem oder mehreren mittelkettigen Fettsäureresten, ein Antiklumpmittel und bis zu 2 Gew.-% Wasser, bezogen auf die Gesamtzusammensetzung, enthält, und das homogene Dispergieren des mindestens einen pharmazeutischen Wirkstoffs in mikronisierter Form in der flüssigen Phase, wobei der mindestens eine mikronisierte pharmazeutische Wirkstoff Bendroflumethiazid oder Amlodipin oder ein pharmazeutisch akzeptables Salz davon ist.

## Revendications

1. Composition pharmaceutique versable adaptée à l'administration par voie orale, cette composition renfermant au moins un agent pharmaceutiquement actif sous forme micronisée dispersé dans une phase liquide qui renferme au moins un triglycéride d'au moins un reste d'acide gras à chaîne moyenne, un agent anti-mottant et jusqu'à 2% en poids d'eau, basé sur la composition globale, l'agent pharmaceutiquement actif micronisé étant le bendroflumethiazide ou l'amlodipine, ou l'un de ses sels pharmaceutiquement acceptables.

2. Composition pharmaceutique conforme à la revendication 1,
dans laquelle la phase liquide est essentiellement exempte d'eau.

3. Composition pharmaceutique conforme à la revendication 1 ou à la revendication 2,
dans laquelle la phase liquide est sous forme monophasée.

4. Composition pharmaceutique conforme à l'une quelconque des revendications précédentes,
dans laquelle l'agent pharmaceutiquement actif est présent à une concentration de 0,001 % w/w à 5% w/w basée sur le total de la composition, de sorte que cet agent pharmaceutiquement actif soit présent à une concentration de 0,1% w/w à 1% w/w basée sur le total de la composition.

5. Composition pharmaceutique conforme à l'une quelconque des revendications précédentes,
dans laquelle la phase liquide renferme en outre au moins un composant liquide hydrophile choisi parmi les composants suivants : glycérine, α-propylène glycol, β-propylène glycol, et polyéthylène glycol.

6. Composition pharmaceutique conforme à l'une quelconque des revendications précédentes,
dans laquelle le triglycéride d'au moins un reste d'acide gras à chaîne moyenne est présent à une concentration de 1% w/w à 99% w/w basée la totalité de la composition, notamment 10% w/w à 99% w/w basée sur le total de la composition.

7. Composition pharmaceutique conforme à la revendication 5 ou à la revendication 6,
dans laquelle le composant liquide hydrophile est présent dans une plage de concentration de 10% w/w à 99% w/w basé le total de la composition.

8. Composition pharmaceutique conforme à l'une quelconque des revendications précédentes,
dans laquelle l'agent pharmaceutiquement actif a un diamètre de particules situé dans la plage de 2 nm à 1 µm, et en particulier l'agent pharmaceutiquement actif a un diamètre de particules situé dans la plage de 100 nm à 0,5 µm.

9. Composition pharmaceutique conforme à l'une quelconque des revendications précédentes,
dans laquelle l'agent anti-mottant est du dioxyde de silicium colloïdal.

10. Composition pharmaceutique conforme à l'une quelconque des revendications précédentes,
destiné à être utilisé dans le cadre d'une thérapie.

11. Procédé de préparation d'une composition pharmaceutique conforme à l'une quelconque des revendications 1 à 9,
comprenant des étapes consistant à se procurer une phase liquide renfermant au moins un triglycéride d'au moins un reste d'acide gras à chaîne moyenne, un agent anti-mottant, et jusqu'à 2% w/w en poids d'eau basé sur le total de la composition, et disperser de manière homogène l'agent pharmaceutiquement actif sous forme micronisée dans la phase liquide, l'agent pharmaceutiquement actif micronisé étant du bendrofluméthiazide ou de la amlodipine ou l'un de ces sels pharmaceutiquement acceptables.
